# EUROPÄISCHE PATENTSCHRIFT

(11) **EP 1 381 686 B1**
(45) Veröffentlichungstag und Bekanntmachung des Hinweises auf die Patenterteilung: **26.07.2006**
(21) Anmeldenummer: 02737952.8
(22) Anmeldetag: 13.04.2002
(51) Int. Cl.: C12N 15/56, C12N 9/24, C12P 9/00, A61K 31/661, A61K 31/665

(54) **INAKTIVIERUNG VON GENEN DES MEP-WEGS**
INACTIVATION OF GENES OF THE MEP PATHWAY
INACTIVATION DE GENES DE LA VOIE MEP

(30) Priorität: 23.04.2001 DE 10119905
(43) Veröffentlichungstag der Anmeldung: 21.01.2004
(73) Patentinhaber: BioAgency AG, 22525 Hamburg (DE)
(72) Erfinder: JOMAA, Hassan, 35392 Giessen (DE); EBERL, Matthias, 35390 Giessen (DE); ALTINCICEK, Boran, 35463 Fernwald- Annerod (DE)
(74) Vertreter: Becker, Philippe
(86) Internationale Anmeldenummer: PCT/EP2002/004134
(87) Internationale Veröffentlichungsnummer: WO 2002/095011

(56) Entgegenhaltungen:
- BORAN ALTINCICEK ET AL.: "Cutting edge: Human gamma/delta T cells are activated by intermediates of the 2-C-methyl-D-erythritol 4-phosphate pathway of isoprenoid biosynthesis" JOURNAL OF IMMUNOLOGY, Bd. 1666, Nr. 6, März 2001 (2001-03), Seiten 3655-3658, XP002230345 in der Anmeldung erwähnt
- CHRISTIAN BELMANT ET AL.: "3-Formyl-1-butyl pyrophosphate: a novel mycobacterial metabolite-activating human gamma-delta T cells " THE JOURNAL OF BIOLOGICAL CHEMISTRY, Bd. 274, Nr. 45, 5. November 1999 (1999-11-05), Seiten 32079-32084, XP002225541 in der Anmeldung erwähnt
- HASSAN JOMAA ET AL.: "Vgamma9/Vdelta2 T cell activation induced by bacterial low molecular mass compounds depends on the 1-deoxy-D-xylulose 5-phosphate pathway of isoprenoid biosynthesis" FEMS IMMUNOLOGY AND MEDICAL MICROBIOLOGY, Bd. 25, 1999, Seiten 371-378, XP002231008
- HINTZ M ET AL: "Identification of (E)-4-hydroxy-3-methyl-but-2-enyl pyrophosphate as a major activator for human gammadelta T cells in Escherichia coli" FEBS LETTERS, ELSEVIER SCIENCE PUBLISHERS, AMSTERDAM, NL, Bd. 509, Nr. 2, 7. Dezember 2001 (2001-12-07), Seiten 317-322, XP004313213 ISSN: 0014-5793
- FEURLE JULIANE ET AL.: "Escherichia coli produces phosphoantigens activating human gammadelta T cells" JOURNAL OF BIOLOGICAL CHEMISTRY, Bd. 277, Nr. 1, 4. Januar 2002 (2002-01-04), Seiten 148-154, XP002230344
- SEEMANN M ET AL: "Isoprenoid biosynthesis via the methylerythritol phosphate pathway: accumulation of 2-C-methyl-d-erythritol 2,4-cyclodiphosphate in a gcpE deficient mutant of Escherichia coli" TETRAHEDRON LETTERS, ELSEVIER SCIENCE PUBLISHERS, AMSTERDAM, NL, Bd. 43, Nr. 5, 28. Januar 2002 (2002-01-28), Seiten 775-778, XP004332805 ISSN: 0040-4039

## Beschreibung

Die vorliegende Erfindung beschreibt Zellen und Organismen, bei denen durch Deletion bzw. Inaktivierung von Genen Intermediate des Mevalonat-unabhängigen Isoprenoid-Stoffwechselwegs (MEP-Weg) angereichert werden. Außerdem beschreibt sie Verfahren zur Herstellung von Intermediaten und abgeleiteten Produkten des MEP-Wegs aus Organismen, bei denen die erfindungsgemäßen Gene deletiert oder inaktiviert worden sind sowie gentechnologische und konventionelle Verfahren zur Herstellung dieser Organismen. Ebenso beschreibt sie die Verwendung von Enzyminhibitoren zur Anreicherung von Intermediaten des MEP-Wegs. Weiterhin beschreibt sie die therapeutische Verwendung von Zellen und Organismen, bei denen die erfindungsgemäßen Gene oder Enzyme deletiert, bzw. inhibiert worden sind und die Herstellung von Arzneistoffen aus diesen Zellen und Organismen.

Die Biosynthese von Isoprenoiden über den klassischen Acetat / Mevalonat-Weg (*Beytia ED, Porter JW. Annu Rev Biochem. 1976;45:113-42*) und einen alternativen, Mevalonat-unabhängigen Biosyntheseweg, den 2-Methyl-D-erythritol-Weg (MEP-Weg, synonym DOXP-Weg) ist bekannt (*Rohmer M. Nat Prod Rep. 1999 Oct;16(5):565-74).* Beide Wege führen zu Isopentenylpyrophosphat (IPP), dem gemeinsamen Vorläufer aller höheren Isoprenoide. Während der Acetat / Mevalonat-Weg seit längerem bekannt und vollständig aufgeklärt ist, sind derzeit noch nicht alle biosynthetischen Reaktionsschritte des MEP-Wegs bekannt.

In der Vergangenheit wurden verschiedene biotechnologische Verfahren, die auf der Anwendung von Erkenntnissen über den MEP-Weg beruhen, abgeleitet:
1. Inhibitoren von verschiedenen Enzymen des MEP-Wegs eignen sich als Antiinfektiva und Herbizide, da der MEP-Weg nicht beim Menschen vorkommt.
2. Bestimmte Intermediate des MEP-Wegs führen zu einer massiven Stimulation von humanen gamma / delta-T-Zellen. Diese Intermediate eignen sich als immonomodulatorische Arzneistoffe.
3. Durch Überexpression bestimmter Gene des MEP-Wegs (z.B. DOXP-Synthase, LytB) konnte eine Anreicherung von höheren Isoprenen als Folgeprodukte des MEP-Wegs erreicht werden.

Bisher unbekannt war jedoch, daß durch Deletion eines Gens des MEP-Wegs, bzw. Inaktivierung des entsprechenden Enzyms die Anreicherung eines Intermediats des MEP-Wegs erreicht werden kann.

Es ist bekannt, daß humane gamma / delta-T-Zellen durch ein oder mehrere Intermediate des MEP-Wegs aktiviert werden. Dies bedeutet, daß es bei Inkubation von peripheren Blutlymphozyten mit Extrakten aus Organismen, die den MEP-Weg besitzen, zu einer selektiven Proliferation und Zytokinsekretion der gamma / delta-T-Zellpopulation kommt *(Jomaa H, Feurle J, Luhs K, Kunzmann V, Tony HP, Herderich M, Wilhelm M. FEMS Immunol Med Microbiol. 1999 Sep;25(4):371-8*). Die genaue chemische Beschaffenheit dieser aktivierenden Substanz oder Substanzen ist noch unbekannt. Publizierte Daten sprechen dafür, daß 3-Formyl-1-Butylpyrophosphat als hypothetisches Intermediat des MEP-Wegs eine Rolle bei der Aktivierung von gamma / delta-T-Zellen spielt (*Belmant C, Espinosa E, Poupot R, Peyrat MA, Guiraud M, Poquet Y, Bonneville M, Fournie JJ. J Biol Chem. 1999 Nov 5;274(45):32079-84*).

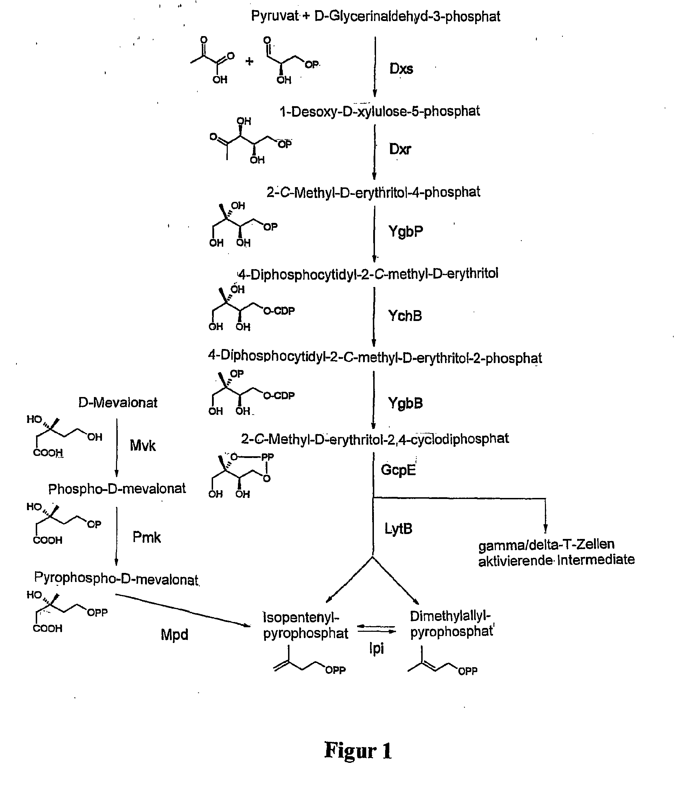

Folgerichtig konnte gezeigt werden, daß Bakterien, bei denen verschiedene Gene des MEP-Wegs (z.B. DOXP-Reductoisomerase, gcpE) deletiert worden waren, nicht mehr in der Lage waren gamma / delta-T-Zellen zu aktivieren (*Altincicek B, Moll J, Campos N, Foerster' G, Beck E, Hoeffler JF, Grosdemange-Billiard C, Rodriguez-Concepcion M, Rohmer M, Boronat A, Eberl M, Jomaa H. J Immunol. 2001 Mar 15;166(6):3655-8*). Zur Herstellung derartiger Deletionsmutanten ist es notwendig, Gene des Mevalonat-Wegs gentechnisch in die Bakterien einzuführen. Dadurch können die Bakterien in Gegenwart von Mevalonat im Medium auch dann überleben, wenn der MEP-Weg nicht mehr funktional ist (Figur 1).

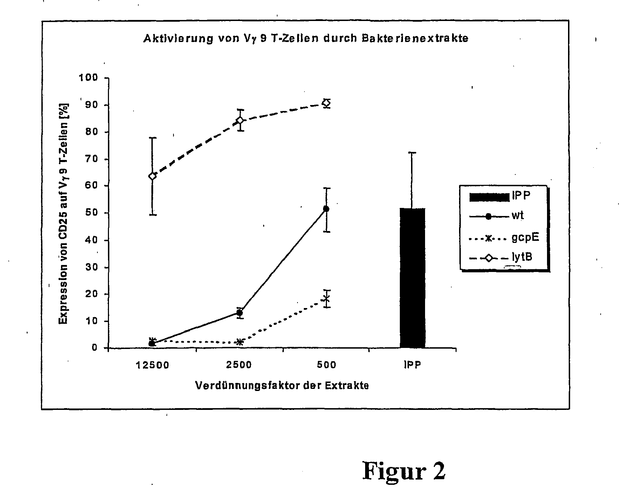

Überraschender Weise hat sich gezeigt, daß Bakterien, deren lytB-Gen deletiert worden war (Beispiel 1), signifikant stärker gamma / delta-T-Zellen aktivierten als wildtypische Bakterien (Beispiel 2, Figur 2). Eine essentielle Beteiligung des lytB-Gens am MEP-Weg konnte gezeigt werden (Beispiel 3). Eine Blockade des MEP-Wegs auf der Höhe des LytB-Enzyms führt somit zur Anreicherung eines Intermediats, das für die gamma / delta-T-Zell-Aktivierung verantwortlich ist.

Es wird daher ein Verfahren zur Verfügung gestellt, bei dem eine Anreicherung von Intermediaten des MEP-Wegs durch Deletion, Mutation oder funktionale Inaktivierung der entsprechenden Gene erreicht wird. Ebenso kann die Anreicherung von Intermediaten des MEP-Wegs durch Inhibition der enzymatischen Funktion der entsprechenden Polypeptide erreicht werden.

Zur Durchführung des erfindungsgemäßen Verfahrens eignen sich besonders DNA-Sequenzen, die für ein Polypeptid mit der in SEQ ID NO:2 dargestellten Aminosäurensequenz codieren oder für ein Analoges oder Derivat des Polypeptids gemäß SEQ ID NO:2, worin eine oder mehrere Aminosäuren deletiert, hinzugefügt oder durch andere Aminosäuren substituiert worden sind, ohne die enzymatische Wirkung des Polypeptids wesentlich zu reduzieren.

Die Erfindung ist ferner durch die Ansprüche 1-7 definiert. Weiterbildungen der Erfindung sind durch die Unteransprüche definiert.
Im folgenden wird die Erfindung anhand der beigefügten Figuren beispielhaft erläutert. Es zeigen:
Figur 1 das Prinzip der Anreicherung von Intermediaten des MEP-Wegs durch Deletion von Genen des MEP-Wegs. Wesentliche Schritte des natürlicherweise in E. coli vorkommenden MEP-Wegs mit den Enzymen Dxs, Dxr, YgbP, YchB, YgbB, Gcpe, LytB sind dargestellt. Um Gene des MEP-Wegs deletieren zu können, wurden Gene des Mevalonatwegs (codierend für Mvk, Pmk, Mpd) durch gentechnologische Methoden eingeführt. Durch Deletion von lytB kommt es zur Anreicherung von Intermediaten, die gamma / delta-T-Zellen aktivieren,
Figur 2 die Aktivierung von gamma / delta-T-Zellen aus dem Blut gesunder Spender, gemessen als Expression von CD25, durch Extrakte verschiedener Bakterienstämme (Wildtyp, wtDelta*gcpE*, wtDelta*lytB*) in verschiedenen Verdünnungsstufen. Als Kontrolle diente IPP bei einer Endkonzentration von 10 uM (IPP aktiviert gamma / delta-T-Zellen wesentlich schwächer als die erfindungsgemäßen Intermediate, eignet sich aber als Kontrolle für das Versuchssystem). Die wtDelta*gcpE*-Mutante wurde analog der wtDelta*lytB*-Mutante hergestellt.

Die Gene und ihre Genprodukte (Polypeptide) sind im Sequenzprotokoll mit ihren Primärstrukturen aufgeführt und haben folgende Zuordnung:
- SEQ ID NO:1: lytB-Gen
- SEQ ID NO:2: lytB-Protein
Die Sequenzen stammen aus Escherichia coli.

Außer den im Sequenzprotokoll genannten DNA-Sequenzen sind auch solche geeignet, die infolge der Degeneration des genetischen Codes eine andere DNA-Sequenz besitzen, jedoch für das gleiche Polypeptid oder für ein Analoges oder Derivat des Polypeptids codieren. Darunter sind auch Sequenzen zu verstehen, die aus anderen Organismen als E. coli, speziell weiteren Bakterien, Algen, Pflanzen und Protozoen, stammen und aufgrund von Sequenzvergleichen oder Funktionsanalysen als homolog zu den im Sequenzprotokoll genannten Sequenzen erkannt werden.

Die Erfindung beschreibt Zellen und Organismen sowie die Herstellung von Zellen und Organismen, bei denen die erfindungsgemäßen Gene durch prinzipiell bekannte Methoden funktional inaktiviert ist. Auch können die Gene nicht vollständig inaktiviert, sondern in ihre Funktion reduziert oder modifiziert sein. Dies kann erreicht werden durch:
- Vollständige oder teilweise Deletion der Gene
- Substitution der Gene durch eine künstliche DNA-Sequenz oder ein Gen für einen Selektionsmarker
- Insertion eines Gens für einen Selektionsmarker
- Deletion, Insertion und Substitution eines oder mehrerer Basenpaare
- Mutationen im 5'- und 3'-Bereich der codierenden Sequenzen (Beeinflussung von Promotor-, Enhancer-, Terminator-Sequenzen, Ribosomen-Bindestellen)
- Einführen von DNA-Konstrukten, die für Antisense-RNA codieren
- Anwendung von mutagenen Agentien, ionisierender Strahlung, UV-Strahlung
- Screening nach Spontanmutanten

Die Inaktivierung oder Modifizierung der erfindungsgemäßen Sequenzen kann bei Bakterien, Algen, Pflanzen und Protozoen erfolgen. Zum Erhalt stabiler Mutanten kann es notwendig sein, die Gene des Acetat / Mevalonat-Wegs teilweise oder vollständig einzuführen und ggf. Mevalonat oder andere Intermediate des Acetat / Mevalonat-Wegs dem Medium zuzusetzen. Alternativ oder zusätzlich können Intermediate des MEP-Wegs oder Derivate oder Analoga dieser Intermediate (z.B. 3-Methyl-3-Buten-1-ol, 3-Methyl-2-Buten-1-ol) dem Medium zugesetzt werden.

Auch können solche Zellen und Organismen verwendet werden, die den MEP-Weg nicht natürlicherweise besitzen, wenn Gene des MEP-Wegs durch gentechnische und biotechnologische Methoden eingeführt worden sind. Dabei ist es auch möglich, die Anreicherung von Intermediaten des MEP-Wegs oder deren Derivate dadurch zu erreichen, dass die Gene des MEP-Wegs nur unvollständig eingeführt werden. Dafür eignen sich u.a. Säugetier- und Insektenzellen, niedere und höhere Pilze, Schleimpilze und verschiedene Protozoen.

Außer durch genetische Methoden kann eine Inaktivierung oder Verringerung der enzymatischen Aktivität der erfindungsgemäßen Polypeptide durch Enzyminhibitoren, die dem Kulturmedium der Organismen oder Zellextrakten aus den Organismen zugesetzt werden, erreicht werden. Bei den Enzyminhibitoren kann es sich um synthetische oder natürliche Verbindungen handeln, die durch kompetitive oder allosterische Wechselwirkungen die Funktion des Polypeptids reversibel oder irreversibel inhibieren.

Die hier beschriebenen Zellen und Organismen, darunter auch vollständige Pflanzen und Teile von Pflanzen, können durch an sich bekannte Verfahren vermehrt und kultiviert werden. Auch ist eine Co-Kultur mit weiteren Zellen oder Organismen, darunter auch solche, die den MEP-Weg nicht besitzen, möglich. Die angereicherten Intermediate des MEP-Wegs oder deren Derivate können durch Aufschluß der Zellen oder aus dem Kulturüberstand gewonnen werden. Zur Aufreinigung der Intermediate eignen sich verschiedene bekannte Methoden, u.a. Chromatogrphie, Elektrophorese und Fällung (z.B. als Bariumsalze).

Die angereicherten Intermediate des MEP-Wegs sind für verschiedene Anwendungen geeignet. Es hat sich herausgestellt, daß die Intermediate das Produkt des GcpE-Enzyms und das Substrat des LytB-Enzyms enthalten. Deshalb können die Intermediate als Substrat in Enzym-Aktivitätstest für LytB und GcpE verwendet werden. Im Aktivitätstest für GcpE wird dabei die Rückreaktion betrachtet. Derartige Enzym-Aktivitätstest eignen sich zum Auffinden von Inhibitoren von GcpE und LytB.

Die angereicherten Intermediate des MEP-Wegs können auch zur Herstellung von Arzneistoffen verwendet werden. Die Wirksamkeit der Substanzen beruht auf der Aktivierung von gamma/delta-T-Zellen. Abhängig von Anwendungsbereich kann dadurch die Immunabwehr gestärkt werden (z.B. gegen Tumore) oder eine immunologische Toleranz gegen Autoantigene und Allergene induziert werden.

Anwendungsbereiche sind die Behandlung von Immun-, Autoimmunerkrankungen und Allergien. Beispiele hierfür sind: Allergien, Multiple Sklerose, Rheumatoide Arthritis, Hashimoto-Thyreoiditis, Myasthenia Gravis, Lupus Erythematodes, Diabetes Mellitus, primärbiliäre Zirrhose, aktive chronische Hepatitis, Adrenalinitis/Addison-Krankheit, Polymyositis, Dermatomyositis, Autoimmunhämolytische Anämie, Herzmuskel- und Herzhautentzündungen, Sklerodermie, Uveitis (Phakouveitis, sympathische Opthalmie), Pemphigus Vulgaris, Pemphigoid, Perniziöse Anämie, Autoimmune atrophische Gastritis, entzündliche Erkrankungen des Darms wie Crohn-Krankheiten und Colitis Ulcerosa, entzündliche Erkrankungen der Lunge wie Asthmatische Erkrankungen und Bronchitiden.

Bevorzugt ist die Anwendung bei Morbus Crohn, Colitis Ulcerosa, Multipler Slderose, Asthma, chronischer Bronchitis, Allergien.

Weitere Anwendungen sind Knochenerkrankungen, insbesondere Osteoporose.

Zur arzneilichen Anwendung können die Intermediate des MEP-Wegs aus den hier beschriebenen Organismen isoliert oder als Rohextrakte verwendet werden. Auch können die kompletten Organismen lebend oder abgetötet verwendet werden. Die hier beschriebenen Intermediate und Organismen könne allein oder in Kombinatiom mit weiteren Arzneistoffen eingesetzt werden. Dabei ist auch die Anwendung als Adjuvant zur Verstärkung oder Modulation einer Immunantwort eingeschlossen. Bevorzugte Anwendungsformen sind die orale, inhalative und rektale Applifikation sowie das Auftragen auf Haut oder Schleimhäute.

Als pharmazeutische Zusammensetzungen eignen sich: Tabletten, Dragees, Kapseln, Pillen, Granulate, Suppositorien, Lösungen, Suspensionen und Emulsionen, Pasten, Salben, Gele, Cremes, Lotionen, Puder und Sprays.

Tabletten, Dragees, Kapseln, Pillen und Granulate können die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumcarbonat, (e) Lösungsverzögerer, z. B. Paraffin und (f) Resorptionsbeschleuniger, z. B. quartemäre Ammonium-verbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe. Ferner können die hier beschriebenen. Verbindungen in andere Trägermaterialien wie zum Beispiel Kunststoffe, (Kunststoffketten zur lokalen Therapie), Kollagen oder Knochenzement eingearbeitet werden.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie die Wirkstoffe nur oder bevorzugt in einem bestimmten Teil des Intestinaltraktes gegebenenfalls verzögert abgeben, wobei als Einbettungsmassen z. B. Polymersubstanzen und Wachse verwendet werden können.

Die Wirkstoffe können gegebenenfalls mit einem oder mehreren der oben angegebenen Trägerstoffe auch in mikroverkapselter Form vorliegen.

Suppositorien können neben den Wirkstoffen die üblichen wasserlöslichen oder wasserunlöslichen Trägerstoffe enthalten, z. B. Polyethylenglykole, Fette, z. B. Kakaofett und höhere Ester (z. B. C14-Alkohol mit C16-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe, enthalten.

Lösungen und Emulsionen können neben den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfu-rylalkohol, Polyethylenglykole und Fettsäureester des Sorbitans oder Gemische dieser Stoffe enthalten.

Von Vorteil ist insbesondere die Wahl einer arzneilichen Darreichungsform, die zusätzlich eine Substanz enthält, welche vom Immunsystem als Fremd- oder Autoantigen erkannt werden kann.

### Beispiel 1

### Konstruktion einer lytB-Deletionsmutante

### Konstruktion des Gen-Austausch-Plasmids pKO3-ΔlytB

Zur Herstellung einer lyt-B-Deletionsmutante von *E*. *coli* wurde der pK03 Vektor benutzt (Link, A. J.; Phillips, D.; Church, G. M.; J. Bacteriol. 179, 6228-6237). Zur Herstellung des Deletionskonstrukts wurden zwei Sequenzen stromabwärts und stromaufwärts des lytB-Gens in zwei asymmetrischen PCR-Ansätzen amplifiziert. Die Primer wurden in 1 : 10- molarem Verhältnis (50 nM und 500 nM) eingesetzt. Beide PCR-Produkte wurden in einer zweiten PCR-Amplifikation zu einem Produkt fusioniert. Das Produkt wurde mit Hilfe des pCR-TA-TOPO Cloning Kits (Invitrogen) kloniert und über die Restriktionsschnittstellen Bam HI und Sal I in den pKO3-Vektor umkloniert. Folgende Primer wurden verwendet:
lytB-N-out, 5'-TAGGATCCccggcctacagattgctgcg-3'
lytB-N-in, 5'-**CCCATCCACTAAACTTAAACA**caacaggatctgcatgttacg-3'
lytB-C-in, 5'-**TGTTTAAGTTTAGTGGATGGG**cgtgaagtcgattaagtcat-3'
lytB-C-out, 5'-TAGTCGACagaaccacccatgatcacc-3'.
Die Restriktionsschnittstellen sind unterstrichen. Überlappende Sequenzen, die eine 21 bp-'in frame'-Insertion definieren, sind fett gedruckt.

### Konstruktion des synthetischen Mevalonat-Operons pSC-MVA

Um Mutanten herstellen zu können, bei denen einzelne Gene des MEP-Wegs deletiert sind, wurde zunächst ein gentechnisch veränderter *E. coli*-Stamm hergestellt, der in der Lage ist, Mevalonat aus dem Kulturmedium für die Synthese von IPP zu nutzen. Dazu wurde ein synthetisches Operon konstruiert, das die Gene für folgende Enzyme des Mevalonat-Wegs aus *Saccharomyces cerevisiae* (Bäckerhefe) enthält: Mevalonat-Kinase (ERG12), Phosphomevalonat-Kinase (ERG8) und Diphosphomevalonat-Decarboxylase (ERG19). Die drei Gene wurden in drei asymmetrische PCR-Ansätzen mit genomischer Hefe-DNA als Matrix amplifiziert, wobei die Primer in 1 : 10- molarem Verhältnis (50 nM und 500 nM) eingesetzt wurden. Mit den Primem wurden Ribosomen-Bindestellen eingeführt. Die drei PCR-Produkte wurden gemischt, so daß sie mit den überlappenden Bereichen hybridisieren konnten, und unter Verwendung der äußeren Primer als ein Fragment amplifiziert. Das Produkt wurde in den pBAD-Vektor unter Verwendung des pBAD-TA-TOPO Cloning Kits (Invitrogen) kloniert und durch Restriktions- und Sequenz-Analyse verifiziert. Das folgende Primer-Set wurde verwendet:
Mev-kin-Sc-for: 5'-T*AGGAGG*AATTAACCATGTCATTACCGTTCTTAACT-3'
Mev-kin-Sc-rev: 5'-**TTGATCTG*CCTCCT*ATGAAGT**CCATGGTAAATT-3'
Pmev-kin-Sc-for: 5'-**ACTTCAT*AGGAGG*CAGATCA**AATGTCAGAGTTGAGAGCCTTC-3'
Pmev-kin-Sc-rev: 5'-**GAGTATTA*CCTCCT*ATTTATC**AAGATAAGTTTC-3'
Decarb-Sc-for: 5'-**GATAAAT*AGGAGG*TAATACTC**ATGACCGTTTACACAGCATCC-3'
Decarb-Sc-rev: 5'-TTATTCCTTTGGTAGACCAGT-3'
Überlappende Sequenzen sind fett gedruckt und Sequenzen, die Ribosomen-Bindestellen definieren, kursiv.
Um die Funktionalität des Operons zu überprüfen, wurde die Sensitivität gegen Fosmidomycin von Bakterien, die mit dem synthetischen Operon transformiert worden waren, in Gegenwart von Mevalonat getestet. Erwartungsgemäß wuchsen Bakterien die das Operon enthielten unter Fosmidomycin mit reduzierter Wachstumsrate, solange das Medium Mevalonat enthielt. Ohne Mevalonat starben die Bakterien unter Fosmidomycin.

### Konstruktion der Deletionsmutante wtΔlytB

Das Plasmid pKO3-Delta*lytB* wurde in den *E. coli* K-12 Stamm DSM No. 498 (ATCC 23716) transformiert, der zuvor mit pSC-MVA, transformiert worden war. Dabei wurde das Medium mit 100 uM Mevalonate supplementiert. Nach 1 h Inkubation bei 30°C wurden Bakterien mit integriertem Plasmid durch einen Temperatur-Shift auf 43°C selektioniert. Durch folgende Testung auf Sucrose-Resistenz und Chloramphenicol-Sensitivität wurden Bakterien, die die Vektorsequenzen verloren hatten, selektioniert und anschließend durch PCR auf den gewünschten Genotyp analysiert.

### Beispiel 2

### Aktivierung von gamma / delta-T-Zellen durch augereicherte Intermediate des MEP-Wegs

Die Anreicherung von Intermediaten des MEP-Wegs wurde über die Fähigkeit dieser Intermediate, gamma / delta-T-Zellen zu aktivieren detektiert. Verschiedene Bakterienstämme (Wildtyp, wtDeltagcpE, wtDeltalytB) wurden in Flüssigkulturen bis zu einer optischen Dichte von ca. 0,8 kultiviert. Die Gewinnung niedermolekularer Extrakte (low molecular weight, LMW) mit einer Ausschlußgrenze von 3 kDa erfolgte wie beschrieben (*Jomaa, H., J. Feurle, K. Luhs, V. Kunzmann, H. P. Tony, M. Herderich, and M. Wilhelm. 1999. FEMS Immunol. Med. Microbiol. 25:371*). Lymphozyten wurden durch Ficoll-Dichtegradienten-Zentrifugation aus dem peripheren Blut von drei gesunden Spendern gewonnen. Für jeden Test wurden 2 × 10⁵ der so erhaltenen Zellen in einem Volumen von 0,2 ml RPMI-1640-Medium (Life Technologies) ausgesät, das mit 25 mM HEPES, 2 mM L-Glutamin, 0,025 mg/ml Gentamycin, 100 U/ml humanes Interleukin-2 (IL-2) (alle von Life Technologies), und 10 % humanem AB-Serum (Bayerisches Rotes Kreuz) angereichert war. LMW-Präparationen wurden in verschiedenen Verdünnungen zugesetzt, als Positivkontrolle wurde IPP (Sigma) in einer Endkonzentration von 10 uM verwendet. Die Inkubation erfolgte bei 37°C und 5 % CO₂ im Brutschrank. Nach 72 Stunden wurden die Zellen geerntet und in einem Durchflußzytometer analysiert. Dabei wurde die Expression des Aktivierungsmarkers CD25 auf der Oberfläche von V gamma 9⁺ T-Zellen gemessen, mit Hilfe der monoklonalen Antikörper CD25-PE (B1.49.9), V gamma 9-FITC (Immu360) und CD3-PC5 (UCHT1) der Firma Beckman-Coulter. Extrakte des Wildtyp-Bakterienstamms aktivierten die gamma / delta-T-Zellen bei einer Verdünnung von 1:500 (entspr. ca. 2 x 10⁷ Bakterien/ml). Extrakte der Delta*gcpE*-Deletionsmutante führten zu einer signifikant geringeren Aktivierung. Dagegen war die Aktivierung durch Extrakte der Delta*lytB*-Deletionsmutante erheblich stärker als durch Extrakte des Wildtyp-Stamms. Eine signifikante gamma / delta-T-Zell-Aktivierung war noch bei einer Verdünnung von 1:12500 (entspr. ca. 8 × 10⁵ Bakterien/ml) zu messen. (Figur 2)

### Beispiel 3

### Beteiligung von IvtB am MEP-Weg

Alle erhaltenen lytB-Deletionsmutanten wuchsen strikt Mevalonat-abhängig. Um diese Beobachtung genauer zu untersuchen, wurde die Deletionsmutante wtDelta*lytB* durch ein wildtypisches lytB-Gen auf einem Plasmid komplementiert. Dazu wurde das lytB-Gen mit den Primem Eclytbfor (5'-GGATCCATGCAGATCCTGTTGGCCAAC-3') und Eclytbrev (5'-AAGCTTTTAATCGACTTCACGAATATCG-3') von genomischer *E*. *coli*-DNA amplifiziert und in den pCR2.1-TOPO-Veldor kloniert. Das Insert wurde über die Restriktionsschnittstellen BamHI und HindIII und in den Expressionsvektor pQE30 umkloniert. Bakterien des Stamms wtDelta*lytB*, die mit diesem Konstrukt transformiert worden waren, konnten wieder ohne Mevalonat wachsen. Dieses Ergebnis bestätigt, daß lytB essentiell am MEP-Stoffwechselweg beteiligt ist. Die angereicherten Intermediate stammen daher tatsächlich aus dem MEP-Weg.

## Patentansprüche

1. Verwendung in vitro von Intermediaten erhältlich durch ein Verfahren **dadurch gekennzeichnet,daß** in ausgewählten Zellen und Organismen das lytB-gen des MEP-Wegs deletiert oder inaktiviert oder derart verändert wird, daß die enzymatische Aktivität des Genprodukts reduziert wird, zur Aktivierung von gamma/delta T-Zellen.

2. Verwendung von Intermediaten erhältlich durch ein Verfahren **dadurch gekennzeichnet,daß** in ausgewählten Zellen und Organismen das lytB-gen des MEP-Wegs delctiert oder inaktiviert oder derart verändert wird, daß die enzymatische Aktivität des Genprodukts reduziert wird , zur Herstellung von Arzneistoffen.

3. Verwendung nach Anspruch 2 zur Herstellung von Arzneistoffen zur Aktivierung von gamma/delta T-Zellen.

4. Verwendung nach einem der Ansprüche 2 oder 3 zur Herstellung von Arzneistoffen zur Behandlung von Erkrankungen bei Mensch und Tier.

5. Verwendung nach einem der Ansprüche 2 bis 4 zur Herstellung von Arzneistoffen zur Behandlung von einer Erkrankung, ausgewählt aus der Gruppe, die aus Asthma, Immun- und Autoimmunerkrankungen, Allergien, Morbus Crohn. Colitis Ulcerosa, Multipler Sklerose, Rheumatoide Arthritis, Hashimoto-Thyreoditis, Myasthenia Gravis, Lupus Erythematodes, Diabetes Mellitus, primär biliäre Zirrhose, aktive chronische Hepatitis, Adrenalinitis/Addison-Krankheit, Polymyositis, Dermatomyositis. Autoimmunhämalytische Anämie, Herzmuskel- und Herzhautentzündungen, Sklerodermie, Uveitis (Phakouveitis, sympathische Opthaklmie), Pemphigus Vulgaris. Pemphigoid, Perniziöse Anämie, und chronischer Bronchitis sowie Knochenerkrankungen, insbesondere Osteoporose, besteht.

6. Verwendung nach Anspruch 5, **dadurch gekennzeichnet, dass** eine arzneiliche Darreichungsform gewählt wird, die zusätzlich eine Substanz enthält, die vom Immunsystem als Fremd- oderAutoantigen erkannt werden kann.

7. Arzneimittel enthaltend ein Intermedial erhältlich durch ein Verfahren **dadurch gekennzeichnet, daß** in ausgewählten. Zellen und Organismen das lytB-gen des MEP-Wegs deletiert oder inaktiviert oder derart verändert wird, daß die enzymatische Aktivität des Genprodukis reduziert wird

## Claims

1. Use *in vitro* of intermediates obtainable by a process, wherein in selected cells and organisms the lytB gene of the MEP pathway is deleted or inactivated or changed such that the enzymatic activity of the gene is reduced for activating gamma/delta T cells.

2. Use of intermediates obtainable by a process, wherein in selected cells and organisms the lytB gene of the MEP pathway is deleted or inactivated or changed such that the enzymatic activity of the gene is reduced for producing medicines.

3. Use according to claim 2 for producing medicines for activating gamma/delta T cells.

4. Use according to claim 2 or 3 for producing medicines for treating human and animal diseases.

5. Use according to anyone of claims 2 to 4 for producing medicines for treating a disease selected from the group comprising asthma, morbus Crohn, colitis ulcerativa, multiple sclerosis, rheumatoid arthritis, Hashimoto's thyroiditis, myasthenia gravis, lupus erythematosus, diabetes mellitus, primary biliary cirrhosis, active chronic hepatitis, adrenalitis/Addison's disease, polymyositis, dermatomyositis, auto-immune haemolysis anemia, myocardial and cardiac infections, scleroderma, uveitis (phacouveitis, sympathic ophtalmia), pemphigus vulgaris, pemphigoid, pernicious anaemia, chronic bronchitis, and bone diseases, especially osteoporosis.

6. Use according to claim 5, wherein the selected drug formulation contains additional substances which can be recognised as foreign or auto-antigen by the immune system.

7. Medicament containing an intermediate obtainable by a process, wherein in selected cells and organisms the lytB gene of the MEP pathway is deleted or inactivated or changed such that the enzymatic activity of the gene is reduced.

## Revendications

1. Utilisation *in vitro* des intermédiaires susceptibles d'être obtenus par un procédé **caractérisé en ce que**, dans des cellules et des organismes sélectionnés, le gène lytB de la voie des MEP est supprimé ou inactivé ou modifié de tel sorte que l'activité enzymatique du gène est réduite pour activer des cellules gamma/delta T.

2. Utilisation des intermédiaires susceptibles d'être obtenus par un procédé **caractérisé en ce que**, dans des cellules et des organismes sélectionnés, le gène lytB de la voie des MEP est supprimé ou inactivé ou modifié de tel sorte que l'activité enzymatique du gène est réduite pour produire des médicaments.

3. Utilisation selon la revendication 2 pour produire des médicaments pour activer des cellules gamma/delta T.

4. Utilisation selon la revendication 2 ou 3 pour produire des médicaments pour traiter des maladies humaines ou animales.

5. Utilisation selon l'une quelconque des revendications 2 à 4 pour produire des médicaments pour traiter une maladie sélectionnée parmi le groupe comprenant l'asthme, la maladie de Crohn, la colite ulcéreuse, la sclérose en plaques, la polyarthrite rhumatoïde, la thyroïdite de Hashimoto, le myasthénie gravis, le lupus érythémateux, le diabète sucré, la cirrhose biliaire primitive, l'hépatite chronique active, la maladie d'Addison/adrénalite, la polymyosite, la dermatomyosite, l'anémie hémolytique auto-immune, les infections du myocarde et cardiaque, la sclérodermie, l'uvéite (la phacouvéite, l'ophtalmie sympatique), le pemphigus vulgaire, la pemphigoïde, l'anémie pernicieuse, la bronchite chronique, et les maladies des os, en particulier l'ostéoporose.

6. Utilisation selon la revendication 5, **caractérisée en ce que** la formulation du médicament sélectionnée contient des substances supplémentaires qui peuvent être reconnues comme étrangères ou auto-antigènes par le système immunitaire.

7. Medicament contenant un intermédiaire susceptible d'être obtenu par un procédé **caractérisé en ce que**, dans des cellules et des organismes sélectionnés, le gène lytB de la voie des MEP est supprimé ou inactivé ou modifié de tel sorte que l'activité enzymatique du gène est réduite.


### SEQUENZPROTOKOLL

<110> Jomaa Pharmaka GmbH
<120> Inaktivierung von Genen des MEP-Wegs
<130> 16819
<140>
   <141>
<150> DE10119905
   <151> 2001-04-23
<160> 2
<170> PatentIn Ver. 2.1
<210> 1
   <211> 951
   <212> DNA
   <213> Escherichia coli
<400> 1
   atgcagatcc tgttggccaa cccacgtggt ttttgtgccg gggtagaccg cgctatcagc 60
   attgttgaaa acgcgcttgc catttacggc gcaccgatat atgtccgtca cgaagtggtg 120
   cataaccgct acgtggtcga tagcctgcgc gagcgtggag ctatctttat tgagcagatc 180
   agcgaagtgc cggacggcgc gatcctgatc ttctccgcac atggtgtttc tcaggcggta 240
   cgtaacgaag cgaaaagccg tgatttgacg gtattcgacg ccacctgtcc gctggtgacc 300
   aaagtgcata tggaagtcgc ccgcgccagc cgtcgtggcg aagagtctat tctcatcggt 360
   cacgccgggc acccggaagt ggaagggacg atggggcagt acagcaaccc tgaaggggga 420
   atgtatctgg tcgaatcgcc tgacgatgtg tggaaactga cggtcaaaaa cgaagagaag 480
   ctctccttta tgacccaaac cacgctgtcg gtagatgaca cgtctgatgt gatcgacgcg 540
   ctgcgtaaac gcttcccgaa aattgtcggt ccgcgcaaag atgacatctg ctacgccacg 600
   actaaccgtc aggaagcggt acgcgccctg gcagaacagg cggaagttgt gttggtggtc 660
   ggttcgaaaa actcctccaa ctccaaccgt ctggcggagc tggcccagcg tatgggcaaa 720
   cgcgcgtttt tgattgacga tgcgaaagat atccaggaag agtgggtgaa agaggttaaa 780
   tgcgtcggcg tgactgcggg cgcatcggct ccggatattc tggtgcagaa tgtggtggca 840
   cgtttgcagc agctgggtgg tggtgaagcc attccgctgg aaggccgtga agaaaatatt 900
   gttttcgaag tgccgaaaga gctgcgtgtc gatattcgtg aagtcgatta a          951
<210> 2
   <211> 316
   <212> PRT
   <213> Escherichia coli
<400> 2
   Met Gln Ile Leu Leu Ala Asn Pro Arg Gly Phe Cys Ala Gly Val Asp
   1 5 10 15
   Arg Ala Ile Ser Ile Val Glu Asn Ala Leu Ala Ile Tyr Gly Ala Pro
   20 25 30
   Ile Tyr Val Arg His Glu Val Val His Asn Arg Tyr Val Val Asp Ser
   35 40 45
   Leu Arg Glu Arg Gly Ala Ile Phe Ile Glu Gln Ile Ser Glu Val Pro
   50 55 60
   Asp Gly Ala Ile Leu Ile Phe Ser Ala His Gly Val Ser Gln Ala Val
   65 70 75 80
   Arg Asn Glu Ala Lys Ser Arg Asp Leu Thr Val Phe Asp Ala Thr Cys
   85 90 95
   Pro Leu Val Thr Lys Val His Met Glu Val Ala Arg Ala Ser Arg Arg
   100 105 110
   Gly Glu Glu Ser Ile Leu Ile Gly His Ala Gly His Pro Glu Val Glu
   115 120 125
   Gly Thr Met Gly Gln Tyr Ser Asn Pro Glu Gly Gly Met Tyr Leu Val
   130 135 140
   Glu Ser Pro Asp Asp Val Trp Lys Leu Thr Val Lys Asn Glu Glu Lys
   145 150 155 160
   Leu Ser Phe Met Thr Gln Thr Thr Leu Ser Val Asp Asp Thr Ser Asp
   165 170 175
   Val Ile Asp Ala Leu Arg Lys Arg Phe Pro Lys Ile Val Gly Pro Arg
   180 185 190
   Lys Asp Asp Ile Cys Tyr Ala Thr Thr Asn Arg Gln Glu Ala Val Arg
   195 200 205
   Ala Leu Ala Glu Gln Ala Glu Val Val Leu Val Val Gly Ser Lys Asn
   210 215 220
   Ser Ser Asn Ser Asn Arg Leu Ala Glu Leu Ala Gln Arg Met Gly Lys
   225 230 235 240
   Arg Ala Phe Leu Ile Asp Asp Ala Lys Asp Ile Gln Glu Glu Trp Val
   245 250 255
   Lys Glu Val Lys Cys Val Gly Val Thr Ala Gly Ala Ser Ala Pro Asp
               260                 265                 270
   Ile Leu Val Gln Asn Val Val Ala Arg Leu Gln Gln Leu Gly Gly Gly
           275                 280                 285
   Glu Ala Ile Pro Leu Glu Gly Arg Glu Glu Asn Ile Val Phe Glu Val
       290                 295                 300
   Pro Lys Glu Leu Arg Val Asp Ile Arg Glu Val Asp
   305                 310                 315